# EUROPÄISCHE PATENTSCHRIFT

(11) **EP 1 129 055 B1**
(45) Veröffentlichungstag und Bekanntmachung des Hinweises auf die Patenterteilung: **01.10.2003**
(21) Anmeldenummer: 99923481.8
(22) Anmeldetag: 28.04.1999
(51) Int. Cl.: C07B 55/00, C07C 211/00

(54) **VERFAHREN ZUR RACEMISIERUNG VON OPTISCH AKTIVEN AMINEN**
METHOD FOR RACEMIZATION OF OPTICALLY ACTIVE AMINES
PROCEDE DE RACEMISATION D'AMINES OPTIQUEMENT ACTIVES

(30) Priorität: 13.11.1998 DE 19852282; 23.12.1998 DE 19859775
(43) Veröffentlichungstag der Anmeldung: 05.09.2001
(73) Patentinhaber: BASF AKTIENGESELLSCHAFT, 67056 Ludwigshafen (DE)
(72) Erfinder: RIECHERS, Hartmut, D-67435 Neustadt (DE); SIMON, Joachim, D-68161 Mannheim (DE); HÖHN, Arthur, D-67281 Kirchheim (DE); KRAMER, Andreas, D-67251 Freinsheim (DE); FUNKE, Frank, D-67117 Limburgerhof (DE); SIEGEL, Wolfgang, D-67117 Limburgerhof (DE); NÜBLING, Christoph, D-67454 Hassloch (DE)
(86) Internationale Anmeldenummer: EP9902874
(87) Internationale Veröffentlichungsnummer: WO00029357

(56) Entgegenhaltungen:
- DE-A- 2 903 589
- DATABASE WPI Section Ch, Week 7908 Derwent Publications Ltd., London, GB; Class B05, AN 79-14874B XP002112502 & JP 54 005957 A (SUMITOMO CHEM CO LTD), 17. Januar 1979 (1979-01-17)
- DATABASE WPI Section Ch, Week 9424 Derwent Publications Ltd., London, GB; Class B05, AN 94-197043 XP002112503 & JP 06 135906 A (TORAY IND INC), 17. Mai 1994 (1994-05-17)

## Beschreibung

Die vorliegende Erfindung betrifft ein Verfahren zur Racemisierung von optisch aktiven Aminen der Formel I 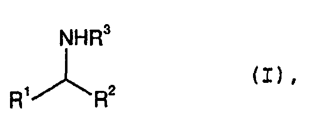 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich für Wasserstoff (H) steht, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins in Gegenwart von Wasserstoff und einem Hydrier- oder Dehydrierkatalysator bei erhöhter Temperatur.

Optisch aktive Amine der Formel I sind z. B. wertvolle Pharmazeutika und Zwischenprodukte zur Herstellung von Wirkstoffen (vergl. z. B.: DE-A-29 03 589, Seite 2, Zeilen 17 bis 26). Da häufig nur eines der beiden Enantiomeren (bezogen auf das in I gezeigte asymmetrische C-Atom) wirksam ist, bzw. wirksamer als das andere Enantiomer ist, werden Verfahren zur Racemisierung des weniger wirksamen Enantiomers, das z. B. bei einer Racematspaltung des entsprechenden racemischen Amins nach bekannten Methoden anfällt, benötigt, weil aus dem racemisierten Amin nach bekannten Methoden (z. B. Racematspaltung) wieder das wirksamere Enantiomer gewonnen werden kann.

IN-A-162 213 (Chem. Abstracts 110: 192247v) offenbart ein Verfahren Herstellung von racemischem 2-Amino-butanol durch Behandlung von *1*-2-Amino-butanol mit Ammoniak in Gegenwart von Rh/Al₂O₃.

US-A-4,096,186 beschreibt ein Verfahren zur Racemisierung von optisch aktiven Aminoalkoholen, wobei der Aminoalkohol, bevorzugt in flüssiger Phase, mit Ammoniak und Wasserstoff in Gegenwart eines Hydrierkatalysators, der bevorzugt Cobalt enthält, in Kontakt gebracht wird. Bei der Umsetzung von optisch aktivem 2-Amino-1-butanol wird bei einer Racematausbeute von maximal 97,6 % nur ein Racemisierungsgrad von 63 % erreicht. Bei einem Racemisierungsgrad von 99 % wird dagegen nur eine Racematausbeute von 75,1 % erreicht.

US-A-4,990,666 offenbart ein Verfahren zur Racemisierung von optisch aktiven Aminoalkoholen, wobei der Aminoalkohol in Gegenwart von Wasserstoff mit Raney-Cobalt in Kontakt gebracht wird. Es wird gelehrt, dass hohe Temperaturen, z. B. größer 160 °C, die Racematausbeute herabsetzen. Weiterhin erfolgt gemäß dieser Lehre die Racemisierung in flüssiger Phase in Gegenwart eines inerten Lösungsmittels.

JP-A-06 135 906 (Derwent Abstract Nr. 94-197043/24; Chem. Abstracts 121: 179093z) beschreibt ein Verfahren zur Racemisierung von optisch aktiven vicinalen primären Diaminen in Gegenwart von Wasserstoff und einem Hydrierkatalysator, wie z. B. Raney-Nickel und Raney-Cobalt.

DE-A-28 51 039 beschreibt ein Verfahren zur Herstellung racemischer Gemische aus optisch aktiven 1-Arylaminen, wobei man die optisch aktiven 1-Arylamine in Gegenwart eines Hydrierkatalysators, insbesondere Raney-Cobalt, in flüssiger Phase mit Wasserstoff behandelt.

DE-A-29 03 589 beschreibt ein Verfahren zur Herstellung racemischer Gemische aus optisch aktiven Aminen, indem man die optisch aktiven Amine mit Wasserstoff in Gegenwart eines Hydrierkatalysators bei erhöhter Temperatur behandelt. Es wird gelehrt, die Racemisierung des optisch aktiven Amins, je nach Beschaffenheit des Amins, in Lösungsmitteln oder in Substanz, d. h. in flüssiger oder fester Form, vorzunehmen. Die Umsetzung von optisch aktivem 2-Amino-1-phenylpropan in flüssiger Phase bei einer Reaktionsdauer von 12 Stunden an einem Raney-Cobalt-Katalysator führt bei einem Racemisierungsgrad von maximal 98 % nur zu einer Racematausbeute von 91,1 %.

Der vorliegenden Erfindung lag die Aufgabe zugrunde, ein verbessertes wirtschaftliches Verfahren zur Racemisierung von optisch aktiven Aminen aufzufinden, bei dem das Verfahrensprodukt mit hohem Racemisierungsgrad bei gleichzeitig hoher Racemisierungsausbeute und hoher Raum-Zeit-Ausbeute erhalten wird.

Demgemäß wurde ein Verfahren zur Racemisierung von optisch aktiven Aminen der Formel I 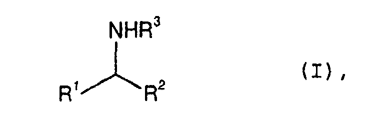 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich für Wasserstoff (H) steht, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins in Gegenwart von Wasserstoff und einem Hydrier- oder Dehydrierkatalysator bei erhöhter Temperatur gefunden, welches dadurch gekennzeichnet ist, dass man die Umsetzung in der Gasphase durchführt.

Das erfindungsgemäße Verfahren läßt sich in der Gasphase diskontinuierlich oder bevorzugt kontinuierlich wie folgt durchführen, wobei der Katalysator bevorzugt als Festbett im Reaktor angeordnet ist.

Das erfindungsgemäße Verfahren kann in Abwesenheit oder bevorzugt in Gegenwart des Amins der Formel R³NH₂, bei dem der Rest R³ dem Rest R³ des optisch aktiven Amins I entspricht, (z. B. des Amins Ammoniak im Fall der Racemisierung von optisch aktiven Aminen I, bei denen R³ = H ist) durchgeführt werden.

Wird in Gegenwart des Amins R³NH₂ gearbeitet, so beträgt im allgemeinen das Molverhältnis von R³NH₂ zu Amin I 1 : 1 bis 50 : 1, bevorzugt 1,5 : 1 bis 30 : 1, besonders bevorzugt 2 : 1 bis 20 : 1, ganz besonders bevorzugt 2 : 1 bis 10 : 1. Der R³NH₂-Überschuß bezogen auf das Amin I kann auch größer als 50 : 1 sein.

Der Wasserstoff wird der Reaktion im allgemeinen in einer Menge von 5 bis 400 l, bevorzugt in einer Menge von 50 bis 200 l, pro Mol Aminkomponente I zugeführt, wobei die Literangaben jeweils auf Normalbedingungen umgerechnet sind (S.T.P.).

Das optisch aktive Amin I wird in einem Rohrreaktor in einem zur Verdampfung ausreichend groß gewählten Gasstrom enthaltend Wasserstoff und vorteilhaft das Amin R³NH₂ bzw. Ammoniak, bevorzugt bestehend aus Wasserstoff und gegebenenfalls dem Amin R³NH₂ bzw. Ammoniak, bei Drücken von 0,1 bis 10 MPa, bevorzugt 0,1 bis 5 MPa, besonders bevorzugt 0,1 bis 3 MPa gasförmig kontinuierlich über den Katalysator geleitet.

Es ist dabei sowohl eine Anströmung des Katalysatorfestbetts von oben als auch von unten möglich. Den erforderlichen Gasstrom erhält man bevorzugt durch eine Kreisgasfahrweise, wobei beispielsweise bei einem Katalysatorschüttvolumen von 1 l eine Kreisgasmenge von ca. 5 bis 10 m³/h (Volumen auf Normalbedingungen umgerechnet) und eine Abgasmenge von ca. 250 bis 350 l/h gefahren wird. Die Katalysatorbelastung liegt im allgemeinen im Bereich von 0,1 bis 2, bevorzugt 0,1 bis 1, besonders bevorzugt 0,2 bis 0,6, kg Amin I pro Liter Katalysator (Schüttvolumen) und Stunde.

Die für die Racemisierung gewählten Temperaturen liegen im Bereich von 100 bis 300 °C, bevorzugt 150 bis 270 °C, besonders bevorzugt 160 bis 250 °C, ganz besonders bevorzugt 170 bis 240 °C, insbesondere bei 180 bis 230 °C.

Die Anwendung höherer Temperaturen, höherer Gesamtdrücke sowie höherer Katalysatorbelastungen als oben angegeben ist möglich. Der Druck im Reaktionsgefäß, welcher sich im wesentlichen aus der Summe der Partialdrücke der Aminkomponente I, des gegebenenfalls vorhandenen Amins R³NH₂ und des gebildeten racemisierten Amins bei der jeweils angewendeten Temperatur ergibt, wird zweckmäßigerweise durch Aufpressen von Wasserstoff auf den gewünschten Reaktionsdruck erhöht.

Aus dem Reaktionsaustrag werden, nachdem dieser zweckmäßigerweise entspannt worden ist, der Wasserstoff und das eventuell verwendete Amin der Formel R³NH₂ entfernt, wobei diese zurückgeführt werden können, und das erhaltene abgekühlte Reaktionsrohprodukt, das im wesentlichen das racemische Amin I enthält, durch eine fraktionierende Rektifikation bei Normaldruck oder bei vermindertem Druck gereinigt.

Als Hydrierkatalysatoren und Dehydrierkatalysatoren sind Katalysatoren, die als katalytisch aktive Bestandteile Elemente, ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, jeweils in metallischer Form (Oxidationsstufe 0) oder in Form von Verbindungen wie z. B. Oxiden, die unter den Verfahrensbedingungen zum entsprechenden Metall reduziert werden, enthalten, besonders geeignet.

Bevorzugt sind Katalysatoren, die als katalytisch aktive Bestandteile Elemente, ausgewählt aus der Gruppe Kupfer, Silber, Cobalt, Nickel, Ruthenium, Rhodium, Palladium, Platin, Chrom und Molybdän, insbesondere ausgewählt aus der Gruppe Kupfer, Silber, Nickel, Ruthenium, Rhodium, Palladium, Chrom und Molybdän, jeweils in metallischer Form (Oxidationsstufe 0) oder in Form von Verbindungen wie z. B. Oxiden, die unter den Verfahrensbedingungen zum entsprechenden Metall reduziert werden, enthalten.

Bevorzugt sind Katalysatoren, die die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram und ein Trägermaterial, ausgewählt aus der Gruppe Aluminiumoxid, Zirkoniumdioxid, Titandioxid, Siliziumdioxid und/oder Kohlenstoff, enthalten.

Die Trägermaterialien Aluminiumoxid (Al₂O₃), Zirkoniumdioxid (ZrO₂), Titandioxid (TiO₂), Siliziumdioxid (SiO₂) und/oder Kohlenstoff sind bei diesen bevorzugt eingesetzten Katalysatoren in der katalytisch aktive Masse im allgemeinen insgesamt in Mengen von 20 bis 99,9 Gew.-%, bevorzugt 30 bis 99,9 Gew.-%, enthalten.

Beispielsweise eignen sich sogenannte Dünnschichtkatalysatoren, bei denen die katalytisch aktiven Komponenten auf strukturierte Träger oder Monolithe, wie sie z. B. in der deutschen Anmeldung Nr. 198 27 385.1 vom 27.06.98, Seite 1, Zeilen 14 bis 30, und in der DE-A-35 13 726 definiert sind, aufgebracht sind. Die katalytisch aktiven Komponenten werden nach bekannten Verfahren auf den verwendeten strukturierten Träger oder Monolith, wie z. B. ein Metalldrahtgewebe oder ein SiO₂-, TiO₂-, ZrO₂- oder Al₂O₃-Wabenkörper, aufgebracht, beispielsweise durch Aufdampfen des katalytisch aktiven Metalls, z. B. Edelmetalls, im Vakuum gemäß der DE-A-35 13 726 oder durch ein Tränkverfahren gemäß der DE-A-41 35 055, DE-A-39 15 685 oder US-A-4,746,537.

Beispiele für im erfindungsgemäßen Verfahren verwendbare Dünnschichtkatalysatoren sind die in EP-A-646 562 in Beispiel 1 und 2 offenbarten Katalysatoren enthaltend den Werkstoff mit der Nr. 1.4767 (Kanthal) und aufgedampftes Pd, der in Beispiel 3 offenbarte Katalysator enthaltend den Werkstoff mit der Nr. 1.4401 und aufgedampftes Pd und der in Beispiel 4 offenbarte Katalysator enthaltend den Werkstoff mit der Nr. 1.4301 und aufgedampftes Pd. (Werkstoffnummern gemäß "Stahleisenliste". Verlag Stahleisen mbH 1990, 8. Aufl., S. 87ff).

Als im erfindungsgemäßen Verfahren verwendbare Hydrier- und Dehydrierkatalysatoren eignen sich weiterhin sogenannte Schalenkatalysatoren, bei denen die katalytisch aktive Masse schalenförmig auf einem Kern von im allgemeinen unter den Reaktionsbedingungen inerten Trägermaterial, wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien, aufgebracht ist.

Zur Herstellung derartiger Schalenkatalysatoren werden üblicher-weise Tränkverfahren eingesetzt, wie sie in J.-F. Le Page et al., Applied Heterogeneous Catalysis, Edition Technip Paris, 1987, ISBN 2-7108-0531-6, Seiten 106 bis 123, beschrieben sind. Diese Tränkverfahren umfassen (a) eine Imprägnierung des Trägermaterials mit einem Überschuß an Lösung (Tauchung) oder (b) eine Sprühimprägnierung des Trägermaterials in einer Tränktrommel sowie jeweils die anschließende Trocknung und Calzinierung.

Eine andere Möglichkeit zur Herstellung derartiger Schalenkatalysatoren ist beispielsweise in DE-A-16 42 938 und DE-A-17 69 998 beschrieben. Danach wird eine wässrige und/oder ein organisches Lösungsmittel enthaltene Lösung oder Suspension der Bestandteile der katalytisch aktiven Masse und/oder deren Vorläuferverbindungen, welche im folgenden als 'Maische' bezeichnet wird, auf das Trägermaterial in einer beheizten Dragiertrommel bei erhöhter Temperatur aufgesprüht, bis der gewünschte Anteil an katalytisch aktiver Masse am Katalysatorgesamtgewicht erreicht ist. Nach DE-A-21 06 796 lässt sich die Beschichtung auch in Wirbelbeschichtern durchführen, wie sie z. B. in der DE-A-12 80 756 beschrieben sind. Die Maische kann dabei gegebenenfalls gemäß der Lehre von EP-A-744 214 organische Binder, bevorzugt Copolymere, wie z. B. Vinylacetat/Vinyllaurat und Vinylacetat/Ethylen, enthalten.

Beispiele für im erfindungsgemäßen Verfahren verwendbare Schalenkatalysatoren sind die in DE-A-20 59 978, Beispiel 1 (Kat. A), offenbarten, durch Tränkung von Tonerde-Agglomeraten mit einer wässrigen Edelmetall-Salzlösung, z. B. Pd-Salzlösung, und anschließender Trocknung und Calcinierung hergestellten, Katalysatoren und die in o.g. Artikel von J.-F. Le Page et al. (Applied Heterogeneous Catalysis) z. B. auf Seite 110 offenbarten, durch Imprägnierung hergestellten und Al₂O₃ und Ni und/oder Co enthaltenden, Katalysatoren.

Im allgemeinen können im erfindungsgemäßen Verfahren die Katalysatoren weiterhin in Form von Katalysatoren, die mittels Tränkungs-, Fällungs- oder Peptisierungsverfahren erhalten wurden, eingesetzt werden und die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Als Träger werden bevorzugt oxidische, carbidische oder nitridische Materialien eingesetzt, besonders bevorzugt Materialien oxidischer Natur.

In diesem Zusammenhang werden als Katalysatorträger verwendete Materialien,
wie Titandioxid (TiO₂; Anatas, Rutil),

Aluminiumoxid (Al₂O₃; bevorzugt α-, β-, γ- oder θ-Al₂O₃; D10-10 von BASF; Al₂O₃ mit großer Oberfläche hergestellt durch in Kontakt bringen mindestens eines Vorläufers von Aluminiumoxid mit mindestens einem Strukturbildner in einem flüssigen Medium, z. B. gemäß der deutschen Anmeldung Nr. 197 30 126.6 vom 14.7.97, auf die hiermit ausdrücklich Bezug genommen wird),

Zirkoniumdioxid (ZrO₂; vorzugsweise in der monoklinen oder tetragonalen Form),

Siliziumdioxid (SiO₂; z. B. über eine Fällung aus Wasserglas oder über das Sol-Gel-Verfahren erhalten oder mesoporöses SiO₂, z. B. mesoporöses SiO₂ mit einer spezifischen Oberfläche der Mesoporen von mindestens 500 m²/g und einem Porenvolumen der Mesoporen von mindestens 1,0 ml/g gemäß DE-A-196 39 016, oder Kieselgel (z. B. nach Ullmann, Enzykl. Techn. Chem., 4. Auflage, Band 21, S. 457-63, 1982) oder in Form von Silikaten, wie Alumosilikaten (z. B. gemäß Nature, Band 359, S. 710-12, 1992 oder Alkali- bzw. Erdalkali-Alumosilikate (Zeolithe), z. B. der allgemeinen Formel M_{2/z}O • Al₂O₃ • xSiO₂ • yH₂O, wobei M ein ein- oder mehrwertiges Metall, H, [NH₄], z die Wertigkeit, x = 1,8 bis ca. 12 und y = 0 bis ca. 8 bedeuten), Magnesiumsilikaten (z. B. Steatit), Zirkoniumsilikaten, Cersilikaten oder Calciumsilikaten) oder SiO₂ mit großer Oberfläche hergestellt durch in Kontakt bringen mindestens eines Vorläufers von Siliciumdioxid mit mindestens einem Strukturbildner in einem flüssigen Medium, z. B. gemäß der deutschen Anmeldung Nr. 197 32 865.2 vom 30.7.97),

Tone, die überwiegend aus Phyllosilikaten und/oder Bandsilikaten bestehen (z. B. Bentonit oder Montmorillonit),

Bims, Siliciumcarbid, Magnesiumoxid (MgO), Zinkoxid (ZnO), Zinndioxid (SnO₂), Cerdioxid (CeO₂), und/oder Kohlenstoff (z. B. Aktivkohle oder Graphit in verstrangter oder tablettierter Form), bzw. deren Gemische, als zur katalytisch aktiven Masse gehörig gewertet.

Die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram sind im allgemeinen insgesamt in Mengen von 0,1 bis 80 Gew.-%, bevorzugt 0,1 bis 70 Gew.-%, besonders bevorzugt 0,1 bis 60 Gew.-%, berechnet als Metall in der Oxidationsstufe 0, in der katalytisch aktiven Masse des Katalysators enthalten.

Die Katalysatoren werden dergestalt eingesetzt, dass man die katalytisch aktive, zu Pulver vermahlene Masse in den Reaktor einbringt oder bevorzugt, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Zur Herstellung dieser Katalysatoren sind verschiedene Verfahren möglich.

Sie sind beispielsweise durch Peptisieren pulvriger Mischungen der Hydroxide, Carbonate, Oxide und/oder anderer Salze der Katalysatorkomponenten mit Wasser und nachfolgendes Extrudieren und Tempern der so erhaltenen Masse erhältlich.

Die im erfindungsgemäßen Verfahren verwendeten Katalysatoren können auch durch Tränkung der Katalysatorträgermaterialien (siehe oben) oder Gemischen zweier oder mehrerer dieser Katalysatorträgermaterialien, die beispielsweise in Form von Pulver oder Formkörpern, wie Strängen, Tabletten, Kugeln oder Ringen, vorliegen, hergestellt werden.

Die Herstellung von Formkörpern der o.g. Katalysatorträgermaterialien kann nach den üblichen Verfahren erfolgen.

Die Tränkung des Katalysatorträgermaterials erfolgt ebenfalls nach den üblichen Verfahren, wie z. B. in EP-A-599 180, EP-A-673 918 oder A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. calziniert.

Die Tränkung kann nach der sogenannten "incipient wetness"-Methode erfolgen, bei der das Katalysatorträgermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calzinieren. Die mehrstufige Tränkung ist vorteilhaft besonders dann anzuwenden, wenn das Katalysatorträgermaterial, mit einer größeren Metallmenge beaufschlagt werden soll.

Zur Aufbringung mehrerer Metallkomponenten auf das Katalysatorträgermaterial kann die Tränkung gleichzeitig mit allen Metallsalzen oder in beliebiger Reihenfolge der einzelnen Metallsalze nacheinander erfolgen. Weiterhin können zur Herstellung der im erfindungsgemäßen Verfahren verwendeten Katalysatoren auch Fällungsmethoden angewendet werden. So können sie beispielsweise durch eine gemeinsame Fällung der Metall-Komponenten aus einer diese Elemente enthaltenden, wässrigen Salzlösung mittels Mineralbasen in Gegenwart einer Aufschlämmung oder Suspension feinkörniger Pulver des schwerlöslichen Katalysatorträgermaterials und anschließendes Waschen, Trocknen und Calcinieren des erhaltenen Präzipitats erhalten werden. Als schwerlösliches Katalysatorträgermaterial kann beispielsweise Aluminiumoxid, Titandioxid, Siliziumdioxid, Zirkoniumdioxid und/oder Zirkoniumoxidhydrat Verwendung finden.

Die im erfindungsgemäßen Verfahren verwendeten Katalysatoren können über eine gemeinsame Fällung (Mischfällung) aller ihrer Komponenten hergestellt werden. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den Katalysatoren weiterverarbeitet. Nach dem Waschen werden sie im allgemeinen bei 80 bis 200 °C, vorzugsweise bei 100 bis 150 °C, getrocknet und danach calciniert. Die Calcinierung wird im allgemeinen bei Temperaturen zwischen 300 und 800°C, vorzugsweise 400 bis 600°C, insbesondere 450 bis 550°C, ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden vor ihrem Einsatz zur Racemisierung der optisch aktiven Amine I üblicherweise vorreduziert. Sie können jedoch auch ohne Vorreduktion eingesetzt werden, wobei sie dann unter den Bedingungen der Racemisierung durch den im Reaktor vorhandenen Wasserstoff reduziert werden.

Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Die folgenden Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile definiert, wobei im Fall der o. g. durch Peptisierung, Tränkung oder Fällung hergestellten Katalysatoren als Katalysatorträger verwendete Materialien zur katalytisch aktiven Masse gehörig gewertet werden.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A, II A, III A, IV A, V A, VI A, I B, II B, III B, IV B und V B des Periodensystems, enthalten.

Beispiele für solche Elemente bzw. deren Verbindungen sind:

Übergangsmetalle, wie Mn bzw. Mn₂O₃ oder MnO₂; V bzw. Vanadiumoxide oder Vanadylpyrophosphat; Nb bzw. Nioboxide oder Nioboxalat; Ta bzw. Tantaloxide; Lanthanide, wie Ce bzw. CeO₂ oder Pr bzw. Pr₂O₃; Alkalimetalloxide, wie Na₂O; Alkalimetallcarbonate; Erdalkalimetalloxide, wie MgO, CaO, SrO, BaO; Erdalkalimetallcarbonate, wie MgCO₃, CaCO₃ und BaCO₃; Boroxid (B₂O₃).

Bevorzugt werden im erfindungsgemäßen Verfahren durch Tränkung, Fällung oder Peptisierung hergestellte Katalysatoren eingesetzt, deren katalytisch aktive Masse vor der Reduktion mit Wasserstoff 20 bis 85 Gew.-%, bevorzugt 25 bis 80 Gew.-%, besonders bevorzugt 30 bis 75 Gew.-%, Aluminiumoxid (Al₂O₃) und/oder Zirkoniumdioxid (ZrO₂) und/oder Titandioxid (TiO₂) und/oder Siliziumdioxid (SiO₂) und/oder Kohlenstoff als Aktivkohle oder Graphit,
1 bis 70 Gew.-%, bevorzugt 2 bis 65 Gew.-%, besonders bevorzugt 4 bis 60 Gew.-%, ganz besonders bevorzugt 20 bis 60 Gew.-%, sauerstoffhaltige Verbindungen des Kupfers, berechnet als CuO,
0 bis 70 Gew.-%, bevorzugt 1 bis 70 Gew.-%, besonders bevorzugt 5 bis 66 Gew.-%, sauerstoffhaltige Verbindungen des Nickels, berechnet als NiO, und
0 bis 50 Gew.-%, bevorzugt 0 bis 30 Gew.-%, beispielsweise 0,1 bis 25 Gew.-%, sauerstoffhaltige Verbindungen des Cobalts, berechnet als CoO, sauerstoffhaltige Verbindungen des Chroms, berechnet als Cr₂O₃, sauerstoffhaltige Verbindungen des Zinks, berechnet als ZnO, sauerstoffhaltige Verbindungen des Molybdäns, berechnet als MoO₃, sauerstoffhaltige Verbindungen des Mangans, berechnet als MnO₂, sauerstoffhaltige Verbindungen des Magnesiums, berechnet als MgO, sauerstoffhaltige Verbindungen des Calciums, berechnet als CaO, und/oder sauerstoffhaltige Verbindungen des Bariums, berechnet als BaO,
enthält.

Die Summer der o.g. Bestandteile der katalytisch aktiven Masse beträgt üblicherweise 70 bis 100 Gew.-%, besonders 80 bis 100 Gew.-%, insbesondere 90 bis 100 Gew.-%, ganz besonders 95 bis 100 Gew.-%, beispielsweise 100 Gew.-%.

Beispiele für solche Katalysatoren sind die in DE-A-19 53 263 offenbarten Katalysatoren enthaltend Cobalt, Nickel und Kupfer und Aluminiumoxid und/oder Siliciumdioxid mit einem Metallgehalt von 5 bis 80 Gew.-%, bezogen auf den gesamten Katalysator, wobei die Katalysatoren, berechnet auf den Metallgehalt, 70 bis 95 Gew.-% einer Mischung aus Cobalt und Nickel und 5 bis 30 Gew.-% Kupfer enthält und wobei das Gewichtsverhältnis von Cobalt zu Nickel 4 : 1 bis 1 : 4 beträgt, beispielsweise die in loc. cit. in den Beispielen beschriebenen Katalysatoren enthaltend 2 bis 4 Gew.-% Kupferoxid, 10 Gew.-% Cobaltoxid und 10 Gew.-% Nickeloxid auf Aluminiumoxid,
die in EP-A-382 049 offenbarten Katalysatoren, deren katalytisch aktive Masse vor der Reduktion mit Wasserstoff 20 bis 85 Gew.-% ZrO₂, 1 bis 30 Gew.-% CuO, und jeweils 1 bis 40 Gew.-% CoO und NiO enthält, beispielsweise die in loc. cit. auf Seite 6 beschriebenen Katalysatoren mit der Zusammensetzung 76 Gew.-% Zr, berechnet als ZrO₂, 4 Gew.-% Cu, berechnet als CuO, 10 Gew.-% Co, berechnet als CoO, und 10 Gew.-% Ni, berechnet als NiO,
die in EP-A-696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält,
beispielsweise der in loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃,
die in der deutschen Anmeldung Nr. 19826396.1 vom 12.06.98 offenbarten Katalysatoren, deren katalytisch aktive Masse vor der Reduktion mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni:Cu-Verhältnis größer 1 ist, 15 bis 50 Gew.-% sauerstoffhaltige Verbindungen des Cobalts, berechnet als CoO, 0 bis 10 Gew.-% sauerstoffhaltige Verbindungen des Aluminiums und/oder Mangans, berechnet als Al₂O₃ bzw. MnO₂, und ) keine sauerstoffhaltigen Verbindungen des Molybdäns enthält,
beispielsweise der in loc. cit., Seite 17, offenbarte Katalysator (A) mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,
die in der deutschen Anmeldung Nr. 19742911.4 vom 29.09.97 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 14 bis 70 Gew.-% sauerstoffhaltige Verbindungen des Nickels, berechnet als NiO, wobei das Ni:Cu-Verhältnis größer 1 ist, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Cobalts oder Molybdäns enthält, beispielsweise der in loc. cit., Seite 14 bis 15, offenbarte Katalysator (A) mit der Zusammensetzung 32 Gew.-% Zr, berechnet als ZrO₂, 51 Gew.-% Ni, berechnet als NiO, und 17 Gew.-% Cu, berechnet als CuO,
die in EP-A-284 919 offenbarten Katalysatoren der allgemeinen Formel MₓMg_{y}(SiO₂)•nH₂O, worin M ein zweiwertiges, reduzierbares Metallatom aus der Gruppe Cu, Fe, Co und Ni, x und y Zahlen sind, die zusammen den Wert 1,5 erreichen können, und n nach Trocknung ausgedrückt in Gew.-% zwischen 0 und 80 liegt, beispielsweise der in loc. cit im Beispiel beschriebene Katalysator enthaltend 35 % CuO, 9 % MgO und 38 % SiO₂ und der in EP-A-863 140 auf Seite 3 beschriebene Katalysator enthaltend 45 bis 47 Gew.-% CuO, Magnesiumsilikat aus etwa 15 bis 17 Gew.-% MgO und 35 bis 36 Gew.-% SiO₂, etwa 0,9 Gew.-% Cr₂O₃, etwa 1 Gew.-% BaO und etwa 0,6 Gew.-% ZnO,
die in DE-A-24 45 303 offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, bei einer Temperatur von 350 bis 700 °C erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird,
die in WO 95/32171 und EP-A-816 350 offenbarten Trägerkatalysatoren enthaltend 5 bis 50, bevorzugt 15 bis 40, Gew.-% Kupfer, berechnet als CuO, 50 bis 95, bevorzugt 60 bis 85, Gew.-% Silicium, berechnet als SiO₂, 0 bis 20 Gew.-% Magnesium, berechnet als MgO, 0 bis 5 Gew.-% Barium, berechnet als BaO, 0 bis 5 Gew.-% Zink, berechnet als ZnO, und 0 bis 5 Gew.-% Chrom, berechnet als Cr₂O₃, jeweils bezogen auf das Gesamtgewicht des calcinierten Katalysators, beispielsweise der in EP-A-816 350, Seite 5, offenbarte Katalysator enthaltend 30 Gew.-% CuO und 70 Gew.-% SiO₂,
die in EP-A-514 692 offenbarten Katalysatoren, deren katalytisch aktive Masse vor der Reduktion mit Wasserstoff 5 bis 100 Gew.-% eines Oxides von Kupfer und Nickel im Atomverhältnis von 1:1 bis 10:1 und Zirkon- und/oder Aluminiumoxid enthält, insbesondere die in loc. cit. auf Seite 3, Zeilen 20 bis 30, offenbarten Katalysatoren, deren katalytisch aktive Masse vor der Reduktion mit Wasserstoff 20 bis 80, besonders 40 bis 70, Gew.-% Al₂O₃ und/oder ZrO₂, 1 bis 30 Gew.-% CuO, 1 bis 30 Gew.-% NiO und gegebenenfalls 1 bis 30 Gew.-% CoO enthält, beispielsweise der in loc. cit., Beispiel 1, beschriebene Katalysator bestehend (nach der Aktivierung) aus 55 Gew.-% Al₂O₃, 36 Gew.-% Cu und 7 Gew.-% Ni,
die in EP-A-691 157 offenbarten Katalysatoren bestehend (vor der Reduktion mit H₂) aus 85 bis 100, insbesondere 95 bis 100, Gew.-% Kupferoxid und Zirkoniumdioxid und 0 bis 15, insbesondere 0 bis 5, Gew.-% Metalloxiden der Nebengruppen Ib bis VIIb und VIII des Periodensystems, beispielsweise der in loc. cit., Seiten 5 bis 6, beschriebene Katalysator mit der Zusammensetzung 52,6 Gew.-% CuO und 47,4 Gew.-% ZrO₂,
die in EP-A-839 575 offenbarten Katalysatoren umfassend, bezogen auf das Gesamtgewicht des Katalysators, mehr als 6 und bis 50 Gew.-% Cobalt, Nickel oder deren Gemisch, 0,001 bis 25 Gew.-% Ruthenium, 0 bis 10 Gew.-% Kupfer und 0 bis 5 Gew.-% Promotoren auf einem porösen Metalloxidträger, wie z. B. Aluminiumoxid, Alumosilikat, Titandioxid, Zirkoniumdioxid, Magnesiumoxid oder Gemischen davon, die durch (a) Imprägnieren des Trägers mit den Metallen, Promotoren oder Verbindungen davon, (b) Trocknen und Calcinieren des imprägnierten Trägers und (c) Reduzieren des calcinierten Trägers im Wasserstoffstrom herstellbar sind,
die in EP-A-839 574 offenbarten Katalysatoren umfassend, bezogen auf das Gesamtgewicht des Katalysators, 0,1 bis 6 Gew.-% Cobalt, Nickel oder deren Gemisch, 0,001 bis 25 Gew.-% Ruthenium, 0 bis 10 Gew.-% Kupfer und 0 bis 5 Gew.-% Promotoren auf einem porösen Metalloxidträger, wie z. B. Aluminiumoxid, Alumosilikat, Titandioxid, Zirkoniumdioxid, Magnesiumoxid oder Gemischen davon, die durch (a) Imprägnieren des Trägers mit den Metallen, Promotoren oder Verbindungen davon, (b) Trocknen und Calcinieren des imprägnierten Trägers und (c) Reduzieren des calcinierten Trägers im Wasserstoffstrom herstellbar sind und
die in der deutschen Anmeldung Nr. 19859776.2 vom 23.12.98 offenbarten Katalysatoren enthaltend Kupfer und sauerstoffhaltige Verbindungen des Titans, wobei der Katalysator in Form von Formkörpern eingesetzt wird, die unter Zusatz von metallischem Kupferpulver hergestellt wurden. Beispielsweise Katalysatoren, deren katalytisch aktive Masse vor der Reduktion mit Wasserstoff 20 bis 83 Gew.-% sauerstoffhaltige Verbindungen des Titans, berechnet als TiO₂, 15 bis 60 Gew.-% sauerstoffhaltige Verbindungen des Kupfers, berechnet als CuO, und 2 bis 29 Gew.-% metallisches Kupfer, das vor der Verformung des Katalysatormaterials zugesetzt wurde, enthalten.

Besonders bevorzugt werden im erfindungsgemäßen Verfahren durch Tränkung, Fällung oder Peptisierung hergestellte Katalysatoren eingesetzt, deren katalytisch aktive Masse weniger als 20 Gew.-%, bevorzugt weniger als 10 Gew.-%, insbesondere weniger als 5 Gew.-%, ganz besonders weniger als 1 Gew.-%, Cobalt, berechnet als CoO, enthält. Ganz besonders bevorzugt enthält die katalytisch aktive Masse keine katalytisch aktiven Mengen an Cobalt oder dessen Verbindungen.

Ganz besonders bevorzugt werden im erfindungsgemäßen Verfahren durch Tränkung, Fällung oder Peptisierung hergestellte Katalysatoren eingesetzt, deren katalytisch aktive Masse vor der Reduktion mit Wasserstoff
20 bis 85 Gew.-%, bevorzugt 25 bis 80 Gew.-%, besonders bevorzugt 30 bis 75 Gew.-%, Aluminiumoxid (Al₂O₃) und/oder Zirkoniumdioxid (ZrO₂) und/oder Titandioxid (TiO₂) und/oder Siliziumdioxid (SiO₂), enthält.

Insbesondere werden Katalysatoren eingesetzt, deren katalytisch aktive Masse vor der Reduktion mit Wasserstoff
35 bis 75 Gew.-% Aluminiumoxid (Al₂O₃),
20 bis 60 Gew.-% sauerstoffhaltige Verbindungen des Kupfers, berechnet als CuO, und
5 bis 45 Gew.-%, bevorzugt 5 bis 20 Gew.-%, sauerstoffhaltige Verbindungen des Nickels, berechnet als NiO,
enthält, wobei die Summe dieser Komponenten mindestens 80 Gew.-%, bevorzugt mindestens 90 Gew.-%, besonders bevorzugt mindestens 95 Gew.-%, beispielsweise 100 Gew.-%, beträgt.
Die Herstellung solcher Katalysatoren kann z. B. gemäß
EP-A-514 692, Seite 3, Zeilen 24 bis 30, erfolgen. Beispielsweise beschreibt loc. cit., Beispiel 1, einen Katalysator bestehend (nach der Aktivierung) aus 55 Gew.-% Al₂O₃, 36 Gew.-% Cu und 7 Gew.-% Ni.

Die Reste R¹, R² und R³ der im erfindungsgemäßen Verfahren eingesetzten optisch aktiven Amine der Formel I 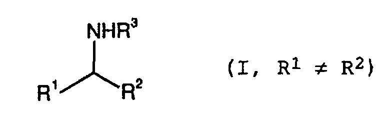 bedeuten unabhängig voneinander Alkyl-, Cycloalkyl-, Arylalkyl-, Aryl-, Heteroarylreste und heterocyclische Reste und R³ zusätzlich Wasserstoff (H), wobei die Reste durch unter den Reaktionsbedingungen inerte Substituenten, ausgewählt aus der Gruppe Alkyl, Cycloalkyl, Alkoxy, Aryloxy, Amino, Alkylamino und Dialkylamino, substituiert sein können, und wobei weiterhin R¹ und R² verschieden sind.

R¹, R² und R³ bedeuten vorzugsweise:
- linearer oder verzweigter Alkylrest, wie C₁₋₂₀-Alkyl, besonders bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl,
   ganz besonders bevorzugt C₁₋₈-Alkyl, wie Methyl, Ethyl, n-Propyl, iso-Propyl, n-Butyl, iso-Butyl, sec.-Butyl, tert.-Butyl und 2-Ethyl-hexyl.
- Cycloalkylrest, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   ganz besonders bevorzugt Cyclopentyl und Cyclohexyl,
- Arylalkylrest, bevorzugt C₇₋₂₀-Arylalkyl, wie Benzyl, 1-Phenethyl, 2-Phenethyl, 1-Naphthylmethyl, 2-Naphthylmethyl, Phenanthrylmethyle, 4-tert.-Butyl-phenyl-methyl, 1-Phenylpropyl, 2-Phenylpropyl, 3-Phenylpropyl, 1-Phenylbutyl, 2-Phenylbutyl, 3-Phenylbutyl und 4-Phenylbutyl,
   besonders bevorzugt Benzyl, 1-Phenethyl und 2-Phenethyl,
- aromatischer Rest, bevorzugt C₆₋₂₀-Aryl, wie Phenyl, 1-Naphthyl, 2-Naphthyl, 1-Anthryl, 2-Anthryl, 9-Anthryl, besonders bevorzugt Phenyl, 1-Naphthyl und 2-Naphthyl, besonders bevorzugt Phenyl,
- heteroaromatischer Rest, bevorzugt C₃₋₁₅-Heteroaryl, wie 2-Pyridinyl, 3-Pyridinyl, 4-Pyridinyl, Chinolinyl, Pyrazinyl, Pyrrol-3-yl, Thienyl, Imidazol-2-yl, 2-Furanyl und 3-Furanyl, und
- heterocyclischer Rest, bevorzugt C₃₋₁₅-Heterocycloalkyl, wie N-Alkyl-piperidin-3-yl, N-Alkyl-piperidin-4-yl, N,N'-Dialkylpiperazin-2-yl, Tetrahydrofuran-3-yl und N-Alkyl-pyrrolidin-3-yl,
wobei in diesen Fällen die Reste R unabhängig voneinander unter den Reaktionsbedingungen inerte Substituenten tragen können, wie z. B. C₁₋₂₀-Alkyl, C₃₋₈-Cycloalkyl, C₁₋₂₀-Alkoxy, C₆₋₂₀-Aryloxy, Amino, C₁₋₂₀-Alkylamino und C₂₋₂₀-Dialkylamino.

Dabei kann die Anzahl dieser Substituenten in R in Abhängigkeit von der Art des Restes 0 bis 5, vorzugsweise 0 bis 3, insbesondere 0, 1 oder 2 betragen. Als Substituenten kommen insbesondere in Betracht:
- C₁₋₂₀-Alkyl, wie oben definiert,
- C₃₋₈-Cycloalkyl, wie oben definiert,
- C₁₋₂₀-Alkoxy, bevorzugt C₁₋₈-Alkoxy, wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy, wie Methoxy, Ethoxy, n-Propoxy, iso-Propoxy, n-Butoxy, iso-Butoxy, sec.-Butoxy und tert.-Butoxy,
- C₆₋₂₀-Aryloxy, wie Phenoxy, 1-Naphthoxy und 2-Naphthoxy, bevorzugt Phenoxy,
- Amino (―NH₂),
- C₂₋₂₀-Dialkylamino, bevorzugt C₂₋₁₂-Dialkylamino, besonders C₂₋₈-Dialkylamino, wie N,N-Dimethylamino, N,N-Diethylamino, N,N-Di-n-propylamino, N,N-Di-iso-propylamino, N,N-Di-n-butylamino, N-Ethyl-N-methylamino, N-Methyl-N-propylamino, Dicyclohexylamino, und
- C₁₋₂₀-Alkylamino, bevorzugt C₁₋₁₂-Alkylamino, besonders C₁₋₈-Alkylamino, wie Methylamino, Ethylamino, n-Propylamino, iso-Propylamino, n-Butylamino, iso-Butylamino, tert.-Butylamino, Cyclopentylamino, Cyclohexylamino.

R³ bedeutet ganz besonders bevorzugt Wasserstoff (H).

Beispiele für im erfindungsgemäßen Verfahren einsetzbare Amine I sind:
1-Methoxy-2-aminopropan (MOIPA), 2-Amino-3-methylbutan,
2-Amino-3,3-dimethylbutan, 1-Phenylethylamin, 1-Naphthylethylamin, 2-Naphthylethylamin, 1-Phenylpropylamin, 2-Amino-1-phenylpropan, 2-Amino-1-(p-hydroxyphenyl)-propan, 2-Amino-1-(p-trifluormethylphenyl)-propan, 2-Amino-1-cyclohexylpropan,
2-Amino-6-methylheptan, 2-Aminoheptan, 2-Amino-4-methylhexan,
1-(4-Methylphenyl)ethylamin, 1-(4-Methoxyphenyl)ethylamin,
1-(3-Methoxyphenyl)ethylamin, 1-Aminotetralin,
trans-1-Amino-2-benzyloxy-cyclopentan und trans-1-Amino-2-benzyloxy-cyclohexan.

Besonders bevorzugt sind 1-Methoxy-2-aminopropan, 2-Amino-3-methylbutan und 2-Amino-3,3-dimethylbutan.

In einer besonderen Variante wird im erfindungsgemäßen Verfahren ein optisch aktives Amin I eingesetzt, das durch Spaltung eines von diesem optisch aktiven Amin abgeleiteten Amids, das bei der Herstellung des entsprechenden Enantiomeren von I (bezogen auf das in I gezeigte asymmetrische C-Atom) durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase und (b) Trennung des resultierenden Gemisches aus optisch aktivem Amin I und Amid anfällt, erhalten wurde.

In einer weiteren besonderen Variante wird im erfindungsgemäßen Verfahren ein optisch aktives Amin I eingesetzt, das bei der Herstellung des entsprechenden Enantiomeren von I (bezogen auf das in I gezeigte asymmetrische C-Atom) durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase, (b) Trennung des resultierenden Gemisches aus optisch aktivem Amin I und Amid und (c) Gewinnung des entsprechenden anderen Enantiomers von I durch Spaltung des Amids erhalten wurde.

Die Verfahren zur Herstellung von optisch aktiven Aminen I aus den entsprechenden Racematen durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase und (b) Trennung des resultierenden Gemisches aus optisch aktivem Amin I und Amid und (c) Gewinnung des entsprechenden anderen Enantiomers von I durch Spaltung des Amids sind in WO 95/08636 und WO 96/23894 beschrieben.

Bei der Hydrolase handelt es sich beispielsweise um eine Lipase, insbesondere eine mikrobielle Lipase. Bei dem Ester handelt es beispielsweise um einen C₁₋₁₂-Alkylester von C₁₋₄-Alkoxyessigsäuren, wie Methoxyessigsäureethylester.

Die Spaltung des vom optisch aktiven Amin I abgeleiteten Amids unter Erhalt der Konfiguration des Chiralitätszentrums kann durch Hydrolyse erfolgen, beispielsweise durch Hydrolyse in Gegenwart eines Polyols oder eines Aminoalkohols und eines Alkali- oder Erdalkalimetallhydroxids gemäß WO 97/10201.

Diese besonderen Verfahrensvarianten gestalten sich besonders ökonomisch, da nach der Herstellung des gewünschten Enantiomers des Amins I, z. B. gemäß WO 95/08636 oder WO 96/23894, das verbliebene, nicht gewünschte Enantiomer von I gemäß den Verfahren dieser Anmeldung racemisiert und erneut in den Verfahren zur Herstellung des gewünschten Enantiomers von I, z. B. gemäß WO 95/08636 oder WO 96/23894, eingesetzt wird. Auf diese Weise wird es möglich, insgesamt mehr als 50 % des gewünschten Enantiomers aus dem racemischen Amin I zu erhalten. (Vergleiche hierzu auch die Ausführungen auf Seite 1 der Beschreibung, 2. Absatz).

### Beispiele

### Beispiel 1:

### Racemisierung von (R)-MOIPA in kontinuierlicher Fahrweise in der Gasphase

(R)-1-Methoxy-isopropylamin ((R)-MOIPA) wurde zusammen mit Ammoniak und Wasserstoff über einen Vorheizer in einen bei 15 bar Überdruck betriebenen Rohrreaktor gefahren. Der Reaktor hatte eine Temperatur von 190 bis 210 °C; die Kreisgasmenge betrug ca. 7 Nm³/(l_{Kat}.*h). Es wurde eine geringe Abgasmenge von 300 Nl/(l_{Kat}.*h) gefahren.

Der Reaktor war mit einem Fällkatalysator der Zusammensetzung 45 Gew.-% CuO, 10 Gew.-% NiO und 45 Gew.-% γ-Al₂O₃-Träger gefüllt. Vor Beginn der Reaktion wurde der Katalysator im Wasserstoffstrom bei 240 °C reduziert. Das Molverhältnis (R)-MOIPA zu Ammoniak betrug 1 : 6, die Katalysatorbelastung lag bei 0,3 kg (R)-MOIPA und 0,29 kg Ammoniak pro Liter Katalysator (Schüttvolumen) und pro Stunde.

Der Reaktoraustrag wurde in einem Abscheider entspannt und destillativ aufgearbeitet.

| GC-Analyse des Austrags (ammoniak- und wasserfrei) in GC-Fl.%: | |
|---|---|
| (R)- + (S)-MOIPA | 92,5 [HPLC: (R)-MOIPA : (S)-MOIPA = 50,3:49,7] |
| Methanol | 0,3 |
| Isopropylamin | 0,6 |
| Methoxyisopropanol | 1,1 |
| Octylamin | 2,1 |
| Octanol | 0,2 |
| Sonstige | 3,2 |
| Racemisierungsgrad: | 99 % |
| Racematausbeute: | 92 % |

### Beispiel 2:

### Racemisierung von (S)-Pinacolylamin ((S)-2-Amino-3,3-dimethylbutan) in kontinuierlicher Fahrweise in der Gasphase

Pinacolylamin (Reinheit: 90 % (S)-Enantiomer, 10 % Pinacolon) wurde zusammen mit Ammoniak und Wasserstoff über einen statischen Mischer und Vorheizer in einen bei Atmosphärendruck betriebenen Rohrreaktor gefahren (Temperatur: 160 bis 200 °C, gerader Durchgang). Der Reaktor war mit Katalysator (Zusammensetzung siehe unten), z. B. 3X3 mm Tabletten, gefüllt. Der Reaktoraustrag wurde kondensiert und zusätzlich Produkt in einer Kühlfalle aufgefangen. Die Analyse des Austrags erfolgte anhand chiraler HPLC.

Die Ergebnisse sind in folgender Tabelle zusammengefasst:

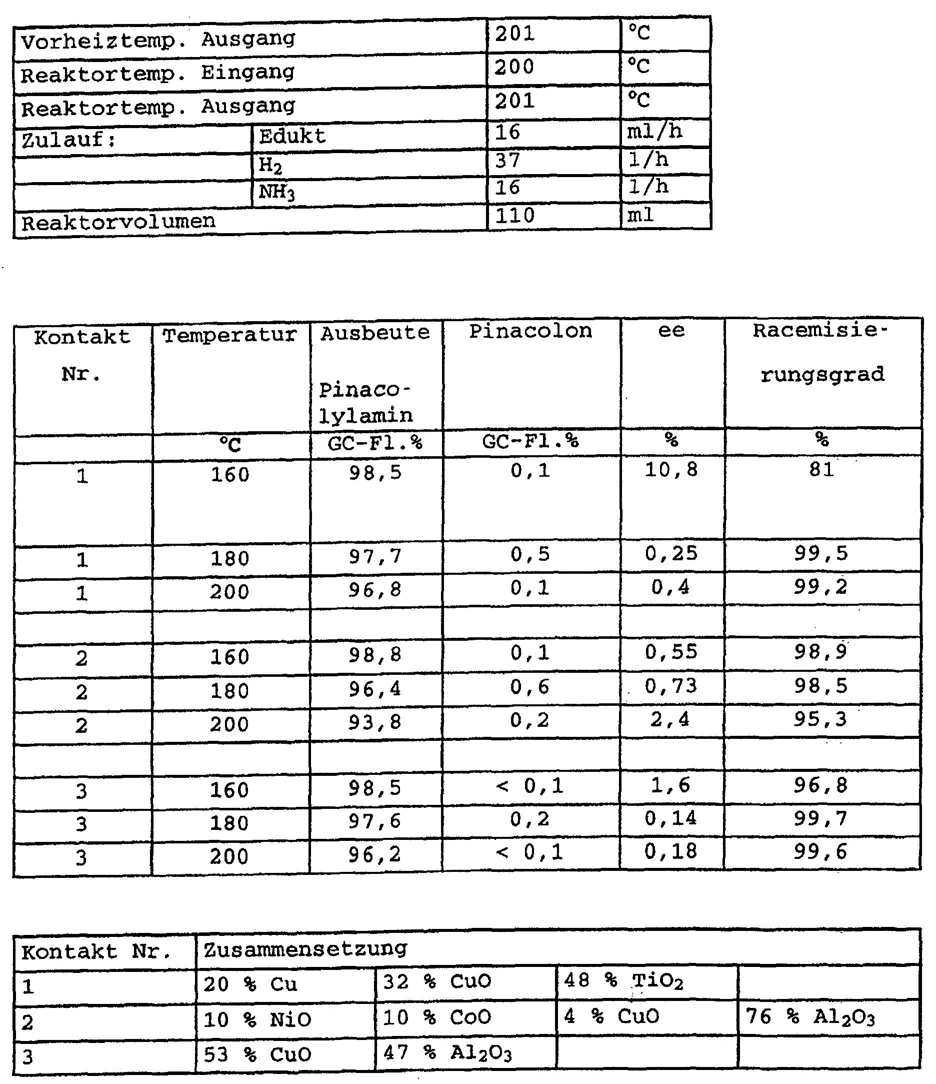

### Vergleichsbeispiel 1:

Versuch zur Racemisierung von (R)-MOIPA analog DE-A-29 03 589 In einem 0,3 1 Autoklaven wurden 10 g (R)-MOIPA (112 mmol), 70 ml Tetrahydrofuran und 1 g Raney-Cobalt vorgelegt. Mit Wasserstoff wurde ein Druck von 20 bar eingestellt. Der Autoklav wurde auf 160 °C aufgeheizt und der H₂-Druck auf 50 bar erhöht. Nach 12 h bei diesen Bedingungen wurde auf Raumtemperatur abgekühlt, der Katalysator abfiltriert und das Tetrahydrofuran am Rotationsverdampfer abgezogen. Die Rückstandsmenge betrug 2,5 g.

Bestimmung des Enantiomerenüberschusses (ee) durch HPLC-Analyse: 4,8 %
(S)-MOIPA = 47,6 Fl.%
(R)-MOIPA = 52,4 Fl.%

| | |
|---|---|
| GC-Analyse [Fl.%]: | |
| Tetrahydrofuran | 0,2 |
| Methoxyisopropanol | 2,2 |
| (R)- + (S)-MOIPA | 68,3 |
| Octylamin | 3,7 |
| Octanol | 10,0 |
| Summe unbek. Verb. | 15,6 |
| Racemisierungsgrad | 90 % |
| Racematausbeute | 61 % |

### Vergleichsbeispiel 2:

### Versuch zur Racemisierung von (S)-3,3-Dimethyl-2-amino-butan (Pinacolylamin) analog DE-A-29 03 589

10 g (S)-Pinacolylamin (99 % ee) wurden zusammen mit 60 g THF und 1 g Raney-Cobalt in einem 0,3 l Rohrautoklaven vermischt und unter Wasserstoffatmosphäre bei einem Druck von 50 bar und einer Temperatur von 165 °C 12 h gerührt.

Dann wurde der Reaktorinhalt auf Raumtemperatur abgekühlt vom Katalysator abgetrennt und das Enantiomerenverhältnis über eine chirale HPLC-Säule bestimmt.
Enantiomerenüberschuss: 99 %
GC-Analyse des Austrags (ammoniak- und wasserfrei) in GC-Fl.%:

| | |
|---|---|
| Pinacolon | 0,1 |
| Pinacolylamin | 99,2 |
| Pinacolol | 0,5 |
| Sonstige | 0,2 |
| Racemisierungsgrad | 0 % |
| Racematausbeute | 99,2 % |

## Patentansprüche

1. Verfahren zur Racemisierung von optisch aktiven Aminen der Formel I 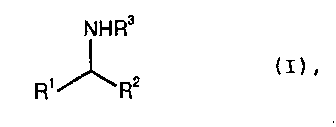 in der R¹ und R² verschieden sind und R¹, R², R³ Alkyl-, Cycloalkyl-, Arylalkyl-, Aryl-, Heteroarylreste und heterocyclische Reste bedeuten und R³ zusätzlich für Wasserstoff steht, wobei die Reste Substituenten, ausgewählt aus der Gruppe Alkyl, Cycloalkyl, Alkoxy, Aryloxy, Amino, Alkylamino und Dialkylamino, tragen können, durch Umsetzung des optisch aktiven Amins in Gegenwart von Wasserstoff und einem Hydrieroder Dehydrierkatalysator bei erhöhter Temperatur, **dadurch gekennzeichnet, dass** man die Umsetzung in der Gasphase durchführt.

2. Verfahren nach Anspruch 1, **dadurch gekennzeichnet, dass** man die Umsetzung in Gegenwart des Amins der Formel R³NH₂, bei dem der Rest R³ dem Rest R³ des optisch aktiven Amins I entspricht, durchführt.

3. Verfahren nach den Ansprüchen 1 und 2, **dadurch gekennzeichnet, dass** man die Umsetzung in Gegenwart eines Katalysators durchführt, der die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram und ein Trägermaterial, ausgewählt aus der Gruppe Aluminiumoxid, Zirkoniumdioxid, Titandioxid, Siliziumdioxid und/oder Kohlenstoff, enthält.

4. verfahren nach den Ansprüchen 1 bis 3, **dadurch gekennzeichnet, dass** die katalytisch aktive Masse des Katalysators die katalytisch aktiven Bestandteile Kupfer, Silber, Gold, Eisen, Cobalt, Nickel, Rhenium, Ruthenium, Rhodium, Palladium, Osmium, Iridium, Platin, Chrom, Molybdän und/oder Wolfram insgesamt in Mengen von 0,1 bis 80 Gew.-% enthält.

5. Verfahren nach den Ansprüchen 1 bis 4, **dadurch gekennzeichnet, dass** die katalytisch aktive Masse des Katalysators ein Trägermaterial, ausgewählt aus der Gruppe Aluminiumoxid, Zirkoniumdioxid, Titandioxid, Siliziumdioxid und/oder Kohlenstoff, insgesamt in Mengen von 20 bis 99,9 Gew.-% enthält.

6. Verfahren nach den Ansprüchen 1 und 2, **dadurch gekennzeichnet, dass** man die Umsetzung in Gegenwart eines Katalysators durchführt, dessen katalytisch aktive Masse vor der Reduktion mit Wasserstoff
20 bis 85 Gew.-% Aluminiumoxid (Al₂O₃), Zirkoniumdioxid (ZrO₂), Titandioxid (TiO₂), Siliziumdioxid (SiO₂) und/oder Kohlenstoff
1 bis 70 Gew.-% sauerstoffhaltige Verbindungen des Kupfers, berechnet als CuO,
0 bis 70 Gew.-% sauerstoffhaltige Verbindungen des Nickels, berechnet als NiO, und
0 bis 50 Gew.-% sauerstoffhaltige Verbindungen des Cobalts, berechnet als CoO, sauerstoffhaltige Verbindungen des Chroms, berechnet als Cr₂O₃, sauerstoffhaltige Verbindungen des Zinks, berechnet als ZnO, sauerstoffhaltige Verbindungen des Molybdäns, berechnet als MoO₃, sauerstoffhaltige Verbindungen des Mangans, berechnet als MnO₂, sauerstoffhaltige Verbindungen des Magnesiums, berechnet als MgO, sauerstoffhaltige Verbindungen des Calciums, berechnet als CaO, und/oder sauerstoffhaltige Verbindungen des Bariums, berechnet als BaO, enthält.

7. Verfahren nach Anspruch 6, **dadurch gekennzeichnet, dass** man die Umsetzung in Gegenwart eines Katalysators durchführt, dessen katalytisch aktive Masse vor der Reduktion mit Wasserstoff
20 bis 85 Gew.-% Aluminiumoxid (Al₂O₃), Zirkoniumdioxid (ZrO₂), Titandioxid (TiO₂) und/oder Siliziumdioxid (SiO₂) enthält.

8. Verfahren nach den Ansprüchen 1 und 2, **dadurch gekennzeichnet, dass** man die Umsetzung in Gegenwart eines Katalysators durchführt, dessen katalytisch aktive Masse vor der Reduktion mit Wasserstoff
35 bis 75 Gew.-% Aluminiumoxid (Al₂O₃),
20 bis 60 Gew.-% sauerstoffhaltige Verbindungen des Kupfers, berechnet als CuO, und
5 bis 45 Gew.-% sauerstoffhaltige Verbindungen des Nickels, berechnet als NiO,
enthält, wobei die Summe dieser Komponenten mindestens
80 Gew.-% beträgt.

9. Verfahren nach den Ansprüchen 1 und 2, **dadurch gekennzeichnet, dass** man die Umsetzung in Gegenwart eines Katalysators durchführt, der, bezogen auf das Gesamtgewicht des Katalysators, mehr als 6 bis 50 Gew.-% Cobalt, Nickel oder deren Gemisch, 0,001 bis 25 Gew.-% Ruthenium, 0 bis 10 Gew.-% Kupfer und 0 bis 5 Gew.-% Promotoren auf einem porösen Metalloxidträger umfasst.

10. Verfahren nach den Ansprüchen 1 und 2, **dadurch gekennzeichnet, dass** man die Umsetzung in Gegenwart eines Katalysators durchführt, der, bezogen auf das Gesamtgewicht des Katalysators, 0,1 bis 6 Gew.-% Cobalt, Nickel oder deren Gemisch, 0,001 bis 25 Gew.-% Ruthenium, 0 bis 10 Gew.-% Kupfer und 0 bis 5 Gew.-% Promotoren auf einem porösen Metalloxidträger umfasst.

11. Verfahren nach den Ansprüchen 1 bis 8, **dadurch gekennzeichnet, dass** man die Umsetzung in Gegenwart eines Katalysators durchführt, dessen katalytisch aktive Masse weniger als 20 Gew.-% Cobalt, berechnet als CoO, enthält.

12. Verfahren nach den Ansprüchen 1 bis 11, **dadurch gekennzeichnet, dass** man die Umsetzung bei Temperaturen von 150 bis 270°C durchführt.

13. Verfahren nach den Ansprüchen 1 bis 12, **dadurch gekennzeichnet, dass** man die Umsetzung bei Drücken von 0,1 bis 5 MPa durchführt.

14. Verfahren nach den Ansprüchen 1 bis 13, **dadurch gekennzeichnet, dass** man als optisch aktives Amin 1-Methoxy-2-aminopropan, 2-Amino-3-methylbutan oder 2-Amino-3,3-dimethylbutan einsetzt.

15. Verfahren nach den Ansprüchen 1 bis 14, **dadurch gekennzeichnet, dass** das optisch aktive Amin I durch Spaltung eines von diesem optisch aktiven Amin abgeleiteten Amids, das bei der Herstellung des entsprechenden Enantiomeren von I durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase und (b) Trennung des resultierenden Gemisches aus optisch aktivem Amin I und Amid anfällt, erhalten wurde.

16. Verfahren nach den Ansprüchen 1 bis 14, **dadurch gekennzeichnet, dass** das optisch aktive Amin I bei der Herstellung des entsprechenden Enantiomeren von I durch (a) enantioselektive Acylierung des racemischen Amins I mit einem Ester, dessen Säurekomponente ein Fluor-, Stickstoff-, Phosphor-, Sauerstoff- oder Schwefelatom in Nachbarschaft des Carbonylkohlenstoffatoms trägt, in Gegenwart einer Hydrolase, (b) Trennung des resultierenden Gemisches aus optisch aktivem Amin I und Amid und (c) Gewinnung des entsprechenden anderen Enantiomers von I durch Spaltung des Amids erhalten wurde.

## Claims

1. A process for the racemization of optically active amines of the formula I 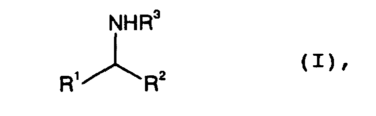 where R¹ and R² are different and R¹, R², R³ are alkyl, cycloalkyl, arylalkyl, aryl, heteroaryl and heterocyclic radicals and R³ can also be hydrogen, where the radicals can bear substituents selected from the group consisting of alkyl, cycloalkyl, alkoxy, aryloxy, amino, alkylamino and dialkylamino, by reaction of the optically active amine in the presence of hydrogen and a hydrogenation or dehydrogenation catalyst at elevated temperature, wherein the reaction is carried out in the gas phase.

2. A process as claimed in claim 1, wherein the reaction is carried out in the presence of the amine of the formula R³NH₂ in which the radical R³ corresponds to the radical R³ of the optically active amine I.

3. A process as claimed in claim 1 or 2, wherein the reaction is carried out in the presence of a catalyst comprising the catalytically active constituents copper, silver, gold, iron, cobalt, nickel, rhenium, ruthenium, rhodium, palladium, osmium, iridium, platinum, chromium, molybdenum and/or tungsten and a support material selected from the group consisting of aluminum oxide, zirconium dioxide, titanium dioxide, silicon dioxide and/or carbon.

4. A process as claimed in any of claims 1 to 3, wherein the catalytically active composition of the catalyst comprises the catalytically active constituents copper, silver, gold, iron, cobalt, nickel, rhenium, ruthenium, rhodium, palladium, osmium, iridium, platinum, chromium, molybdenum and/or tungsten in a total amount of from 0.1 to 80% by weight.

5. A process as claimed in any of claims 1 to 4, wherein the catalytically active composition of the catalyst comprises a support material selected from the group consisting of aluminum oxide, zirconium dioxide, titanium dioxide, silicon dioxide and/or carbon in a total amount of from 20 to 99.9% by weight.

6. A process as claimed in claim 1 or 2, wherein the reaction is carried out in the presence of a catalyst whose catalytically active composition before reduction with hydrogen comprises from 20 to 85% by weight of aluminum oxide (Al₂O₃), zirconium dioxide (ZrO₂), titanium dioxide (TiO₂), silicon dioxide (SiO₂) and/or carbon
from 1 to 70% by weight of oxygen-containing compounds of copper, calculated as CuO,
from 0 to 70% by weight of oxygen-containing compounds of nickel, calculated as NiO, and
from 0 to 50% by weight of oxygen-containing compounds of cobalt, calculated as CoO, oxygen-containing compounds of chromium, calculated as Cr₂O₃, oxygen-containing compounds of zinc, calculated as ZnO, oxygen-containing compounds of molybdenum, calculated as MoO₃, oxygen-containing compounds of manganese, calculated as MnO₂, oxygen-containing compounds of magnesium, calculated as MgO, oxygen-containing compounds of calcium, calculated as CaO, and/or oxygen-containing compounds of barium, calculated as BaO.

7. A process as claimed in claim 6, wherein the reaction is carried out in the presence of a catalyst whose catalytically active composition before reduction with hydrogen comprises from 20 to 85% by weight of aluminum oxide (Al₂O₃), zirconium dioxide (ZrO₂), titanium dioxide (TiO₂) and/or silicon dioxide (SiO₂).

8. A process as claimed in claim 1 or 2, wherein the reaction is carried out in the presence of a catalyst whose catalytically active composition before reduction with hydrogen comprises
from 35 to 75% by weight of aluminum oxide (Al₂O₃),
from 20 to 60% by weight of oxygen-containing compounds of copper, calculated as CuO, and
from 5 to 45% by weight of oxygen-containing compounds of nickel, calculated as NiO,
where the sum of these components is at least 80% by weight.

9. A process as claimed in claim 1 or 2, wherein the reaction is carried out in the presence of a catalyst which, based on the total weight of the catalyst, comprises from > 6 to 50% by weight of cobalt, nickel or a mixture thereof, from 0.001 to 25% by weight of ruthenium, from 0 to 10% by weight of copper and from 0 to 5% by weight of promoters on a porous metal oxide support.

10. A process as claimed in claim 1 or 2, wherein the reaction is carried out in the presence of a catalyst which, based on the total weight of the catalyst, comprises from 0.1 to 6% by weight of cobalt, nickel or a mixture thereof, from 0.001 to 25% by weight of ruthenium, from 0 to 10% by weight of copper and from 0 to 5% by weight of promoters on a porous metal oxide support.

11. A process as claimed in any of claims 1 to 8, wherein the reaction is carried out in the presence of a catalyst whose catalytically active composition contains less than 20% by weight of cobalt, calculated as CoO.

12. A process as claimed in any of claims 1 to 11, wherein the reaction is carried out at from 150 to 270°C.

13. A process as claimed in any of claims 1 to 12, wherein the reaction is carried out at a pressure of from 0.1 to 5 MPa.

14. A process as claimed in any of claims 1 to 13, wherein the optically active amine used is 1-methoxy-2-aminopropane, 2-amino-3-methyl-butane or 2-amino-3,3-dimethylbutane.

15. A process as claimed in any of claims 1 to 14, wherein the optically active amine I has been obtained by cleavage of an amide derived from this optically active amine, which amide is formed in the preparation of one enantiomer of I by (a) enantioselective acylation of the racemic amine I with an ester whose acid component bears a fluorine, nitrogen, phosphorus, oxygen or sulfur atom adjacent to the carbonyl carbon in the presence of a hydrolase and (b) separation of the resulting mixture of optically amine I and amide.

16. A process as claimed in any of claims 1 to 14, wherein the optically active amine I has been obtained in the preparation of one enantiomer of I by (a) enantioselective acylation of the racemic amine I with an ester whose acid component bears a fluorine, nitrogen, phosphorus, oxygen or sulfur atom adjacent to the carbonyl carbon in the presence of a hydrolase, (b) separation of the resulting mixture of optically active amine I and amide and (c) isolation of the other enantiomer of I by cleavage of the amide.

## Revendications

1. Procédé de racémisation d'amines optiquement actives de formule I 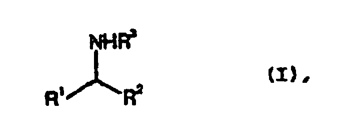 dans laquelle R¹ et R² sont différents et R¹, R², R³ représentent des groupes alkyle, cycloalkyle, arylalkyle, aryle, hétéroaryle et des groupes hétérocycliques et R³ représente en plus un atome d'hydrogène, ces groupes pouvant porter des substituants choisis parmi les groupes alkyle, cycloalkyle, alcoxy, aryloxy, amino, alkylamino et dialkylamino,
par réaction de l'amine optiquement active en présence d'hydrogène et d'un catalyseur d'hydrogénation ou de déshydrogénation, à température élevée, **caractérisé en ce que** la réaction est réalisée en phase gazeuse.

2. Procédé selon la revendication 1, **caractérisé en ce que** la réaction est réalisée en présence de l'amine de formule R³NH₂, dans laquelle le groupe R³ correspond au groupe R³ de l'amine optiquement active I.

3. Procédé selon les revendications 1 et 2, **caractérisé en ce que** la réaction est réalisée en présence d'un catalyseur contenant des composants à action catalytique tels que le cuivre, l'argent, l'or, le fer, le cobalt, le nickel, le rhénium, le ruthénium, le rhodium, le palladium, l'osmium, l'iridium, le platine, le chrome, le molybdène et/ou le tungstène, et un support choisi dans le groupe formé par l'oxyde d'aluminium, le dioxyde de zirconium, le dioxyde de titane, le dioxyde de silicium et/ou le carbone.

4. Procédé selon les revendications 1 à 3, **caractérisé en ce que** la masse à action catalytique du catalyseur contient des composants à action catalytique tels que le cuivre, l'argent, l'or, le fer, le cobalt, le nickel, le rhénium, le ruthénium, le rhodium, le palladium, l'osmium, l'iridium, le platine, le chrome, le molybdène et/ou le tungstène à raison, au total, de 0,1% à 80% en poids.

5. Procédé selon les revendications 1 à 4, **caractérisé en ce que** la masse à action catalytique du catalyseur contient un support choisi dans le groupe formé par l'oxyde d'aluminium, le dioxyde de zirconium, le dioxyde de titane, le dioxyde de silicium et/ou le carbone, à raison, au total, de 20% à 99,9% en poids.

6. Procédé selon les revendications 1 et 2, **caractérisé en ce que** la réaction est réalisée en présence d'un catalyseur dont la masse à action catalytique avant la réduction de l'hydrogène contient :
- 20% à 85% en poids d'oxyde d'aluminium (Al₂O₃), de dioxyde de zirconium (ZrO₂), de dioxyde de titane (TiO₂), de dioxyde de silicium (SiO₂) et/ou de carbone,
- 1% à 70% en poids de composés oxygénés du cuivre, calculé en CuO,
- 1% à 70% en poids de composés oxygénés du nickel, calculé en NiO, et
- 0% à 50% en poids de composés oxygénés du cobalt, exprimé en CoO, de composés oxygénés du chrome, calculé en Cr₂O₃, de composés oxygénés du zinc, calculé en ZnO, de composés oxygénés du molybdène, calculé en MoO₃, de composés oxygénés du manganèse, calculé en MnO₂, de composés oxygénés du magnésium, calculé en MgO, de composés oxygénés du calcium, calculé en CaO, et/ou de composés oxygénés du baryum, calculé en BaO.

7. Procédé selon la revendication 6, **caractérisé en ce que** la réaction est réalisée en présence d'un catalyseur dont la masse à action catalytique avant la réduction par l'hydrogène contient 20% à 85% en poids d'oxyde d'aluminium (Al₂O₃), de dioxyde de zirconium (ZrO₂), de dioxyde de titane (TiO₂) et/ou de dioxyde de silicium (SiO₂).

8. Procédé selon les revendications 1 et 2, **caractérisé en ce que** la réaction est réalisée en présence d'un catalyseur dont la masse à action catalytique avant la réduction par l'hydrogène contient :
- 35% à 75% en poids d'oxyde d'aluminium (Al₂O₃),
- 20% à 60% en poids de composés oxygénés du cuivre, calculé en CuO et
- 5% à 45% en poids de composés oxygénés du nickel, calculé en NiO, la somme de ces composants s'élevant au moins à 80% en poids.

9. Procédé selon les revendications 1 et 2, **caractérisé en ce que** la réaction est réalisée en présence d'un catalyseur comprenant, par rapport au poids total du catalyseur, plus de 6% à 50% en poids de cobalt, de nickel ou de leur mélange, 0,001% à 25% en poids de ruthénium, 0% à 10% en poids de cuivre et 0% à 5% en poids de promoteurs, sur un support d'oxyde de métal poreux.

10. Procédé selon les revendications 1 et 2, **caractérisé en ce que** la réaction est réalisée en présence d'un catalyseur comprenant, par rapport au poids total du catalyseur, 0,1% à 6% en poids de cobalt, de nickel ou de leur mélange, 0,001% à 25% en poids de ruthénium, 0% à 10% en poids de cuivre et 0% à 5% en poids de promoteurs, sur un support d'oxyde de métal poreux.

11. Procédé selon les revendications 1 à 8, **caractérisé en ce que** la réaction est réalisée en présence d'un catalyseur dont la masse à action catalytique contient moins de 20% en poids de cobalt, calculé en CoO.

12. Procédé selon les revendications 1 à 11, **caractérisé en ce que** la réaction est réalisée à une température comprise entre 150°C et 270°C.

13. Procédé selon les revendications 1 à 12, **caractérisé en ce que** la réaction est réalisée à une pression comprise entre 0,1 et 5 MPa.

14. Procédé selon les revendications 1 à 13, **caractérisé en ce que**, en tant qu'amine optiquement active, le 1-méthoxy-2-amino-propane, le 2-amino-3-méthylbutane ou le 2-amino-3,3-diméthylbutane est mis en oeuvre.

15. Procédé selon les revendications 1 à 14, **caractérisé en ce que** l'amine optiquement active I a été obtenue par dissociation d'un amide dérivé de cette amine optiquement active, formé lors de la préparation de l'énantiomère correspondant de l'amine I par :
(a) acylation sélective d'énantiomère de . l'amine racémique I avec un ester dont le composant acide porte un atome de fluor, d'azote, de phosphore, d'oxygène ou de soufre à proximité de l'atome de carbone carbonyle, en présence d'une hydrolase et
(b) séparation du mélange résultant de l'amine optiquement active I et de l'amide.

16. Procédé selon les revendications 1 à 14, **caractérisé en ce que** l'amine optiquement active I a été obtenue lors de la préparation de l'énantiomère correspondant de l'amine I par :
(a) acylation sélective d'énantiomère de l'amine racémique I avec un ester dont le composant acide porte un atome de fluor, d'azote, de phosphore, d'oxygène ou de soufre à proximité de l'atome de carbone carbonyle, en présence d'une hydrolase
(b) séparation du mélange résultant de l'amine optiquement active I et de l'amide et
(c) obtention de l'autre énantiomère correspondant de l'amine I par dissociation de l'amide.
